# EUROPÄISCHE PATENTSCHRIFT

(11) **EP 1 238 639 B1**
(45) Veröffentlichungstag und Bekanntmachung des Hinweises auf die Patenterteilung: **07.06.2006**
(21) Anmeldenummer: 01105644.7
(22) Anmeldetag: 07.03.2001
(51) Int. Cl.: A61F 2/01

(54) **Filtervorrichtung für Gefässsysteme**
Filter device for vessel systems
Dispositif de filtre pour systèmes de vaisseaux

(43) Veröffentlichungstag der Anmeldung: 11.09.2002
(73) Patentinhaber: Bavaria Medizin Technologie GmbH, D-82234 Wessling/Oberpfaffenhofen (DE)
(72) Erfinder: Brahmer, Dieter, 82216 Maisach (DE); Sauerteig, Knut, 86932 Pürgen (DE)
(74) Vertreter: Hofstetter, Alfons J.

(56) Entgegenhaltungen:
- WO-A-00/49970
- WO-A-01/08595
- US-A- 5 910 154

## Beschreibung

Die vorliegende Erfindung betrifft eine Filtervorrichtung für Gefäßsysteme zur Verwendung bei chirurgischen Eingriffen, insbesondere bei minimalinvasiven chirurgischen Eingriffen innerhalb von Gefäßsystemen wie Blutgefäßen, Hohlräumen von Organen und ähnlichem, mit einem länglichen, rohrförmigen Filterkörper und mindestens einem selbständig expandierbaren Fangsack aus einem engmaschigen Geflecht zum Auffangen und Ausfiltern von abgelösten Gewebeteilchen und/oder Gefäßablagerungen, wobei der Fangsack im nicht-expandierten Zustand von einer am Umfang des Filterkörpers verschiebbaren Schutztülle umgeben ist.

Während einer Ballondilatation oder dem Implantieren eines Stents innerhalb eines Gefäßes wie z.B. einem Blutgefäß können sich kleinste Teile der Gefäßablagerungen innerhalb der Blutgefäße lösen und dadurch die Bildung von Thromben verursachen. Insbesondere bei der Behandlung von Koronargefäßen oder bei der Behandlung der Halsschlagader ist dies sehr kritisch, da die durch die Thrombenbildung entstehenden Folgeschäden bis hin zum Tod des Patienten führen können.

Es wurden daher verschiedene Filtervorrichtungen für Gefäßsysteme der eingangs genannten Art entwickelt. So beschreibt z.B. die internationale Patentanmeldung WO 99/25252 eine vaskuläre Filtervorrichtung mit einem länglichen Filterkörper aus einem Geflecht mit hexagonalen Öffnungen, wobei der Filterkörper im expandierten Zustand einen Durchmesser aufweist, der dem Innenumfang des entsprechenden Blutgefäßes, in dem die Filtervorrichtung eingesetzt worden ist, entspricht. Des weiteren sind sogenannte Führungsdraht-Systeme mit Embolie-Schutzfilter bekannt. Dabei weist der Führungsdraht an seiner distalen Spitze den Schutzfilter auf, der aus einer dünnen, porösen Membran über einer sehr dünnen, metallenen Strebenkonstruktion besteht. In nicht-entfaltetem Zustand ist dieser bekannte Filter in einer Entfaltungshülse untergebracht.

Nachteilig an den bekannten Filtervorrichtungen für Gefäßsysteme zur Verwendung bei chirurgischen Eingriffen ist jedoch, dass die expandierbaren oder entfaltbaren Filterelemente nach einem Expandieren oder Entfalten nicht mehr oder nur unter größtem Aufwand in ihre ursprüngliche, nicht-expandierte bzw. nicht-gefaltete Form übergeführt werden können. Dadurch ergeben sich insbesondere bei einer Entfernung der bekannten Filtervorrichtungen aus den Gefäßsystemen erhebliche Probleme, die zu einer erheblichen Komplikation des chirurgischen Eingriffs und damit einer Gefährdung des Patienten führen können.

Aus der US 5,910,154 ist eine Filtervorrichtung für Gefäßsysteme zur Verwendung bei chirurgischen Eingriffen bekannt. Die Filtervorrichtung weist einen Fangsack auf, welcher an einem Außenumfang des Katheters angeordnet ist. Eine Schutztülle ist derart über den Außenumfang des Katheters verschiebbar angeordnet, dass der Fangsack durch das Verschieben der Schutztülle expandierbar oder schließbar ist. Im geschlossenen Zustand, d. h. wenn die Schutztülle über den Fangsack geschoben ist, liegt der geschlossene Fangsack an dem Außenumfang des Katheters an. Die bekannte Filtervorrichtung ist somit nur in Kombination mit einem Katheter realisierbar.

Es ist daher Aufgabe der vorliegenden Erfindung eine Filtervorrichtung für Gefäßsysteme zur Verwendung bei chirurgischen Eingriffen der eingangs genannten Art bereitzustellen, die einerseits eine hohe Filterleistung und andererseits ein leichtes Entfernen aus dem Gefäßsystem gewährleistet. Insbesondere ist es Aufgabe der Erfindung, eine Filtervorrichtung für Gefäßsysteme bei chirurgischen Eingriffen bereitzustellen, mit der auch Gewebeteilchen und Gefäßablagerungen mit geringer Größe sicher ausgefiltert und zurückgehalten werden können.

Zur Lösung dieser Aufgabe dient eine gattungsgemäße Filtervorrichtung gemäß den Merkmalen des Hauptanspruchs 1.

Vorteilhafte Ausgestaltungen sind in den Unteransprüchen beschrieben.

Eine erfindungsgemäße Filtervorrichtung für Gefäßsysteme zur Verwendung bei chirurgischen Eingriffen weist einen Fangsack auf, der mit einem Ende an einem Filterkörper befestigt ist, so dass der Fangsack bei einem Zurückziehen einer Schutztülle sich trichterförmig öffnet und ein dem geschlossenen Ende gegenüberliegendes offenes Ende mit einer Öffnung ausbildet. Dabei ist die Schutztülle derart ausgebildet, dass bei einem Verschieben der Schutztülle in Richtung des offenen Endes die Schutztülle auf den expandierten Fangsack aufgeschoben wird, so dass sich der Fangsack schließt und am Umfang des Filterkörpers anliegt. Durch die vorteilhafte Befestigung des Fangsackes mit nur einem Ende an dem Filterkörper ist gewährteistet, dass sich die trichterförmige Öffnung des Fangsackes über den gesamten Innendurchmesser des Gefäßes, in dem die Filtervorrichtung anliegt, ausdehnt. Somit können in dem Bereich der Filtervorrichtung alle abgelösten Gewebeteilchen und/oder Gefäßablagerungen, die mit dem Blut- oder Flüssigkeitsstrom innerhalb der Gefäße transportiert werden, sicher aufgefangen und ausgefiltert werden. Die Gefahr der Thrombenbildung verringert sich dadurch erheblich. Des weiteren ist gewährleistet, dass die Filtervorrichtung ohne Probleme wieder aus dem Gefäßsystem entfernt werden kann, da die Schutztülle derart ausgebildet ist, dass sie wieder auf den expandierten Fangsack aufgeschoben werden kann und diesen in den nicht-expandierten Zustand überführt. Vorteilhafterweise wird dadurch die Belastung des Patienten durch den chirurgischen Eingriff deutlich verringert. Der Fangsack weist im expandierten Zustand eine Maschenweite zwischen 0,01 und 0,3 mm auf, wobei die maximale Maschenweite im Bereich des offenen Endes erreicht wird. Durch die Verringerung der Maschenweite von dem offenen Ende in Richtung des geschlossenen Endes erhöht sich die Filterwirkung deutlich, da auch Gewebeteilchen und Gefäßablagerungen mit geringer Größe sicher ausgefiltert und zurückgehalten werden.

In einer vorteilhaften Ausgestaltung der Erfindung besteht der Fangsack aus einem Metallgeflecht oder einem Geflecht aus einer Metalllegierung oder einem Kunststoffgeflecht. Besonders vorteilhaft ist, wenn der Fangsack aus einem Nitinolgeflecht besteht, da Nitinol sich durch die Körperwärme automatisch ausdehnt und es so zu einer Selbstexpansion des Fangsackes kommt.

In einer weiteren vorteilhaften Ausgestaltung der erfindungsgemäßen Filtervorrichtung weist die Schutztülle an dem auf den Fangsack aufzuschiebenden Ende eine trichterförmige Erweiterung auf. Zudem kann die Schutztülle flexibel ausgebildet sein. Beide Ausgestaltungen erleichtern vorteilhafterweise das Aufschieben der Schutztülle auf den expandierten Fangsack. Durch die flexible Ausgestaltung der Schutztülle können zudem relativ große Mengen an ausgefiltertem Material in dem Fangsack abgelagert werden, ohne dass das Aufschieben der Schutztülle auf den gefüllten Fangsack beeinträchtigt wird.

In einer weiteren vorteilhaften Ausgestaltung der erfindungsgemäßen Filtervorrichtung ist das Ende des Fangsacks mit dem Filterkörper verklebt oder verschweißt oder mittels einer Befestigungsvorrichtung mit dem Filterkörper verbunden. Dadurch ist eine nicht-lösbare Verbindung zwischen dem einen Ende des Fangsackes und des Filterkörpers gewährleistet.

In einer weiteren vorteilhaften Ausgestaltung der Erfindung ist an dem Filterkörper ein Marker zur Feststellung der Position der Filtervorrichtung innerhalb des Gefäßsystems angeordnet. Dadurch ist eine Kontrollmöglichkeit bezüglich der Position des Filterkörpers bzw. der Filtervorrichtung gegeben, die zur exakten Positionierung der Filtervorrichtung dient.

In einer weiteren vorteilhaften Ausgestaltung der erfindungsgemäßen Filtervorrichtung ist innerhalb des rohrförmigen Filterkörpers ein Führungsdraht geführt. Dadurch ist es möglich, dass die Filtervorrichtung exakt innerhalb des Gefäßsystems positioniert werden kann.

In einer weiteren vorteilhaften Ausgestaltung der erfindungsgemäßen Filtervorrichtung ist der maximale Durchmesser der Filtervorrichtung bei nicht-expandiertem Fangsack derart gewählt, dass die Filtervorrichtung innerhalb eines Katheters geführt werden kann. Dadurch ist es möglich, die erfindungsgemäße Filtervorrichtung in Kombination mit Kathetern einzusetzen. Die Filtervorrichtung kann dabei als eine Art Führungsdraht zur Führung und Platzierung des Katheters verwendet werden.

In weiteren vorteilhaften Ausgestaltungen der erfindungsgemäßen Filtervorrichtung ist diese fest mit einem Ballonkatheter oder mit einem System für selbst-expandierende Stents verbunden. Dadurch ist es möglich, die Anzahl der chirurgischen Eingriffe auf ein Minimum zu reduzieren, da die genannten Gerätekombinationen nur einmal mit einem Einführvorgang in das Gefäßsystem des Patienten eingebracht werden.

Verwendung findet die erfindungsgemäße Filtervorrichtung bei vaskulären, koronaren und kranialen chirurgischen Interventionen bzw. Eingriffen.

Weitere Einzelheiten, Merkmale und Vorteile der Erfindung ergeben sich aus dem in den Figuren beschriebenen und beispielhaft dargestellten Ausführungsbeispiel:

Es zeigen
- Figur 1: eine schematische Darstellung einer erfindungsgemäßen Filtervorrichtung im nicht-expandierten Zustand;
- Figur 2: eine schematische Darstellung der erfindungsgemäßen Filtervorrichtung gemäß Figur 1 im expandierten Zustand;
- Figur 3: eine schematische Darstellung einer erfindungsgemäßen Filtervorrichtung im nicht-expandierten Zustand, die mit einem System für selbst-expandierende Stents verbunden ist; und
- Figur 4: eine schematische Darstellung der erfindungsgemäßen Filtervorrichtung gemäß Figur 3 im expandierten Zustand.

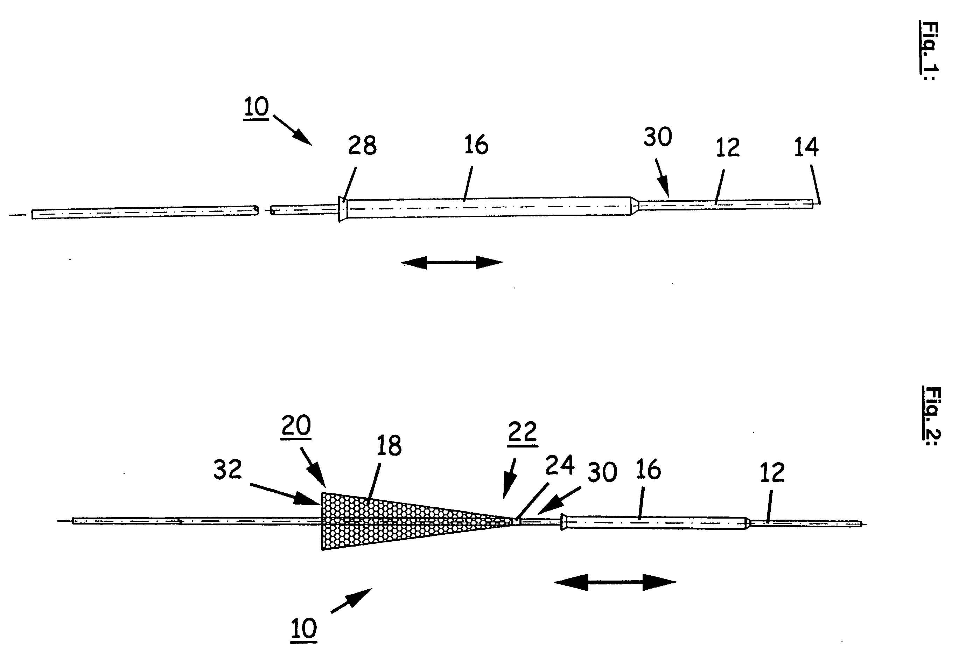

Figur 1 zeigt in einer schematischen Darstellung eine Filtervorrichtung 10 für Gefäßsysteme zur Verwendung bei chirurgischen Eingriffen mit einem länglich, rohrförmigen Filterkörper 12. Am Umfang 30 des Filterkörpers 12 ist eine verschiebbare Schutztülle 16 angeordnet, die einen selbständig expandierbaren Fangsack 18 aus einem engmaschigen Geflecht zum Auffangen und Ausfiltern von abgelösten Gewebeteilchen und/oder Gefäßablagerungen umgibt. Man erkennt, dass die Schutztülle 16 an einem Ende eine trichterförmige Erweiterung 28 aufweist. Die Schutztülle 16 ist zudem üblicherweise flexibel ausgebildet. Des weiteren erkennt man, dass innerhalb des rohrförmigen Filterkörpers 12 ein Führungsdraht 14 geführt ist.

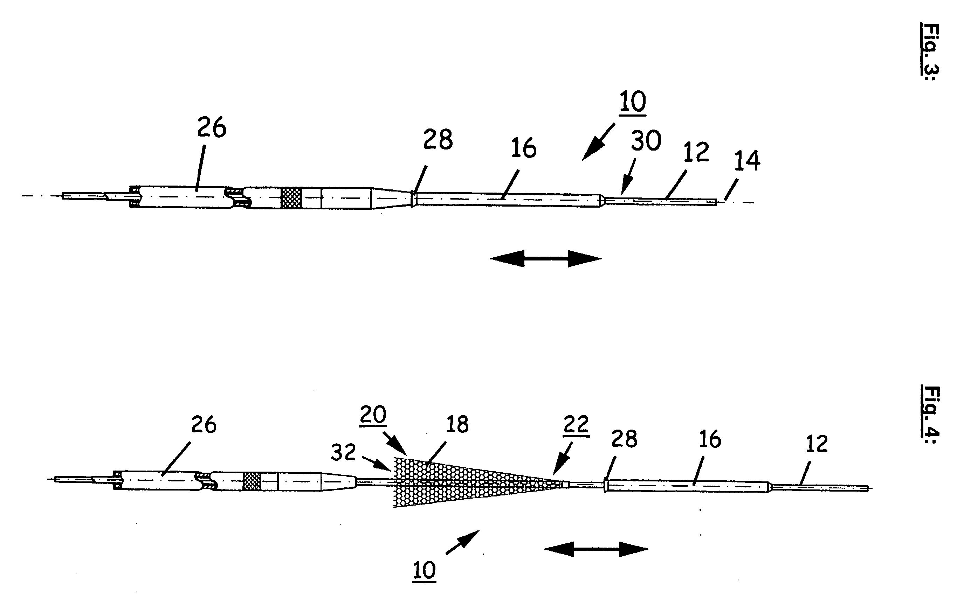

Figur 2 zeigt die Filtervorrichtung 10 mit einem expandierten Fangsack 18. Zur Expansion des Fangsackes 18 wurde die Schutztülle 16 zurückgeschoben, so dass sich der Fangsack 18 entfalten konnte. Man erkennt, dass der Fangsack 18 mit einem Ende 22 an dem Filterkörper 12 befestigt ist, so dass er sich trichterförmig öffnet und ein dem geschlossenen Ende 22 gegenüberliegendes offenes Ende 20 mit einer Öffnung 32 ausbildet. Der Fangsack 18 weist im expandierten Zustand eine Maschenweite zwischen 0,01 und 0,3 mm auf und erreicht die maximale Maschenweite im Bereich des offenen Endes 20. In dem dargestellten Ausführungsbeispiel besteht der Fangsack aus einem selbständig expandierbaren Nitinolgeflecht. Es ist aber auch möglich, dass der Fangsack aus einem Metallgeflecht oder einem Geflecht aus einer anderen Metalllegierung oder einem Kunststoffgeflecht besteht.

Des weiteren erkennt man, dass die Schutztülle 16 derart ausgebildet ist, dass bei einem Verschieben der Schutztülle 16 in Richtung des offenen Endes 20 die Schutztülle 16 auf den expandierten Fangsack 18 aufgeschoben wird, so dass sich der Fangsack 18 schließt und am Umfang 30 des Filterkörpers 12 anliegt. Die trichterförmige Erweiterung 28 an dem auf den Fangsack 18 aufzuschiebenden Ende der Schutztülle 16 erleichtert diesen Vorgang.

Das Ende 22 des Fangsackes 18 kann mit dem Filterkörper 12 verklebt oder verschweißt sein. In dem dargestellten Ausführungsbeispiel ist das Ende 22 mittels einer Befestigungsvorrichtung 24 mit dem Filterkörper 12 verbunden.

Figur 3 zeigt eine weitere Ausführungsform der Filtervorrichtung 10. Man erkennt, dass die Filtervorrichtung 10 fest mit einem System für selbst-expandierende Stents 26 verbunden ist. In Figur 3 ist dabei die Filtervorrichtung 10 im nicht-expandierten Zustand, in Figur 4 im expandierten Zustand, d.h. mit einem expandierten Fangsack 18 dargestellt.

Die Verwendung der erfindungsgemäßen Filtervorrichtung 10 soll im folgenden anhand eines Anwendungsbeispiels erläutert werden.

So kann die Filtervorrichtung 10 z.B. bei der Beseitigung von Stenosen innerhalb von Gefäßen verwendet werden. Dabei wird nach einer angiographischen Untersuchung das entsprechende Gefäß mit der Stenose lokalisiert. Anschließend wird der Führungsdraht 14 in das Gefäß eingeführt und passiert die Stenose. Über den Führungsdraht 14 wird die Filtervorrichtung 10 ebenfalls über die Stenose hinaus vorgeschoben. Anschließend folgt der zum Einsatz kommende Dilatationskatheter oder das Stentträgersystem, die ebenfalls über die Filtervorrichtung bis zum Einsatzort geschoben werden. Nach Verschieben der Schutztülle 16 expandiert der Fangsack 16 selbständig, so dass anschließend die Stenose auf konventionelle Art dilatiert oder ein Stent implantiert werden kann. Etwaige Gefäßablagerungen, die sich während der Dilatation oder der Implantierung des Stents von der Gefäßinnenwand lösen, werden durch den Fangsack 18 der Filtervorrichtung 10 sicher abgefangen. Nach der Beseitigung der Stenose und der Kontrolle des Ergebnisses mittels einer Kontrastmittelinjektion wird die Schutztülle 16 wieder über den Fangsack 18 gezogen. Abschließend erfolgt die Entfernung aller Geräte aus dem Gefäßsystem.

## Patentansprüche

1. Filtervorrichtung für Gefäßsysteme zur Verwendung bei chirurgischen Eingriffen, insbesondere bei minimalinvasiven chirurgischen Eingriffen innerhalb von Gefäßsystemen wie Blutgefäßen, Hohlräumen von Organen und ähnlichem, mit einem länglichen, rohrförmigen Filterkörper (12) und mindestens einem selbständig expandierbaren Fangsack (18) aus einem engmaschigen Geflecht zum Auffangen und Ausfiltern von abgelösten Gewebeteilchen und/oder Gefäßablagerungen, wobei der Fangsack (18) im nicht-expandierten Zustand von einer am Umfang (30) des Filterkörpers (12) verschiebbaren Schutztülle (16) umgeben ist,
**dadurch gekennzeichnet,**
**dass** der Fangsack (18) mit einem Ende (22) an dem Filterkörper (12) befestigt ist, so dass der Fangsack (18) bei einem Zurückziehen der Schutztülle (16) sich trichterförmig öffnet und ein dem geschlossenen Ende (22) gegenüberliegendes offenes Ende (20) mit einer Öffnung (32) ausbildet, wobei die Schutztülle (16) derart ausgebildet ist, dass bei einem Verschieben der Schutztülle (16) in Richtung des offenen Endes (20) die Schutztülle (16) auf den expandierten Fangsack (18) aufgeschoben wird, so dass sich der Fangsack (18) schließt und am Umfang (30) des Filterkörpers (12) anliegt, wobei der Fangsack (18) im expandierten Zustand eine Maschenweite zwischen 0,01 und 0,3 mm aufweist und die maximale Maschenweite im Bereich des offenen Endes (20) erreicht.

2. Filtervorrichtung nach Anspruch 1,
**dadurch gekennzeichnet,**
**dass** der Fangsack (18) aus einem Metallgeflecht oder einem Geflecht aus einer Metalllegierung oder einem Kunststoffgeflecht besteht.

3. Filtervorrichtung nach Anspruch 2,
**dadurch gekennzeichnet,**
**dass** der Fangsack (18) aus einem Nitinolgeflecht besteht.

4. Filtervorrichtung nach einem der vorhergehenden Ansprüche,
**dadurch gekennzeichnet,**
**dass** die Schutztülle (16) an dem auf den Fangsack (18) aufzuschiebenden Ende eine trichterförmige Erweiterung (28) aufweist.

5. Filtervorrichtung nach einem der vorhergehenden Ansprüche,
**dadurch gekennzeichnet,**
**dass** die Schutztülle (16) flexibel ausgebildet ist.

6. Filtervorrichtung nach einem der vorhergehenden Ansprüche,
**dadurch gekennzeichnet,**
**dass** das Ende (22) des Fangsacks (18) mit dem Filterkörper (12) verklebt oder verschweißt ist oder mittels einer Befestigungsvorrichtung (24) mit dem Filterkörper (12) verbunden ist.

7. Filtervorrichtung nach einem der vorhergehenden Ansprüche,
**dadurch gekennzeichnet,**
**dass** an dem Filterkörper (12) ein Marker zur Feststellung der Position der Filtervorrichtung (10) innerhalb des Gefäßsystems angeordnet ist.

8. Filtervorrichtung nach einem der vorhergehenden Ansprüche,
**dadurch gekennzeichnet,**
**dass** innerhalb des rohrförmigen Filterkörpers (12) ein Führungsdraht (14) geführt ist.

9. Filtervorrichtung nach einem der vorhergehenden Ansprüche,
**dadurch gekennzeichnet,**
**dass** der maximale Durchmesser der Filtervorrichtung (10) bei nicht-expandiertem Fangsack (18) derart gewählt ist, dass die Filtervorrichtung (10) innerhalb eines Katheters geführt werden kann.

10. Filtervorrichtung nach einem der vorhergehenden Ansprüche,
**dadurch gekennzeichnet,**
**dass** die Filtervorrichtung (10) fest mit einem Ballonkatheter verbunden ist.

11. Filtervorrichtung nach einem Ansprüche 1 bis 10,
**dadurch gekennzeichnet,**
**dass** die Filtervorrichtung (10) fest mit einem System für selbst-expandierende Stents (26) verbunden ist.

## Claims

1. Filtering device for vascular systems for use in surgical interventions, especially in minimally-invasive surgical interventions within vascular systems such as blood vessels, cavities of organs and the like, including an elongate, tubular filter body (12) and at least one automatically expandable catching bag (18) of a narrow-meshed network for collecting and filtering-out released tissue particles and/or vessel depositions, wherein the catching bag (18) is surrounded by a protecting sleeve (16) displaceable on the periphery (30) of the filter body (12) in the non-expanded state,
**characterized in that**
the catching bag (18) is attached to the filter body (12) at one end (22) such that the catching bag (18) opens funnel-shaped upon retracting the protecting sleeve (16) and forms an open end (20), opposing the closed end (22), with an opening (32), wherein the protecting sleeve (16) is formed such that upon displacing the protecting sleeve (16) towards the open end (20), the protecting sleeve (16) is pushed onto the expanded catching bag (18), such that the catching bag (18) closes and abuts the periphery (30) of the filter body (12), wherein the catching bag (18) has a mesh size of between 0,01 and 0,3 mm in the expanded state, and reaches the maximum mesh size in the region of the open end (20).

2. Filtering device according to claim 1,
**characterized in that**
the catching bag (18) is made of a metal network or of a network of a metal alloy or of a plastic network.

3. Filtering device according to claim 2,
**characterized in that**
the catching bag (18) is made of a nitinol network.

4. Filtering device according to any one of the preceding claims,
**characterized in that**
the protecting sleeve (16) has a funnel-shaped expansion (28) at the end to be pushed onto the catching bag (18).

5. Filtering device according to any one of the preceding claims,
**characterized in that**
the protecting sleeve (16) is flexibly formed.

6. Filtering device according to any one of the preceding claims,
**characterized in that**
the end (22) of the catching bag (18) is adhered or welded to the filter body (12) or is connected to the filter body (12) by means of an attaching device (24).

7. Filtering device according to any one of the preceding claims,
**characterized in that**
a marker for detecting the position of the filtering device (10) within the vascular system is disposed on the filter body (12).

8. Filtering device according to any one of the preceding claims,
**characterized in that**
a guide wire (14) is guided within the tubular filter body (12).

9. Filtering device according to any one of the preceding claims,
**characterized in that**
the maximum diameter of the filtering device (10) with non-expanded catching bag (18) is selected such that the filtering device (10) can be guided within a catheter.

10. Filtering device according to any one of the preceding claims,
**characterized in that**
the filtering device (10) is fixedly connected to a balloon catheter.

11. Filtering device according to any one of claims 1 to 10,
**characterized in that**
the filtering device (10) is fixedly connected to a system for self-expanding stents (26).

## Revendications

1. Dispositif de filtrage de systèmes vasculaires destiné à être utilisé dans des interventions chirurgicales, notamment en cas d'interventions chirurgicales d'invasion minimale à l'intérieur de systèmes vasculaires tels que les vaisseaux sanguins, les cavités d'organes et semblables, comportant un corps de filtre (12) tubulaire oblong et au moins un sachet de captage (18) à auto-dilatation réalisé en un treillis à mailles étroites pour capter et filtrer des particules de tissu et/ou des dépôts vasculaires, le sachet de captage (18) étant entouré, en état non-dilaté, d'une douille protectrice (16) déplaçable sur le contour (30) du corps de filtre (12),
**caractérisé en ce**
**que** le sachet de captage (18) est fixé par une extrémité (22) au corps de filtre (12), de manière que le sachet de captage (18), lors d'une rétraction de la douille protectrice (16), s'ouvre de façon conique pour définir, en vis-à-vis de l'extrémité fermée (22), une extrémité ouverte (20) présentant une ouverture (32), la douille protectrice (16) étant prévue de manière à se glisser, lors d'un déplacement de la douille protectrice (16) en direction de l'extrémité ouverte (20), sur le sachet de captage (18) dilaté de manière à obturer ce dernier (18) et l'amenant à épouser le contour (30) du corps de filtre (12), le sachet de captage (18) présentant, en état dilaté, un maillage compris entre 0,01 et 0,3 mm, tout en atteignant le maillage maxi. au droit de l'extrémité ouverte (20).

2. Dispositif de filtrage selon la revendication 1,
**caractérisé en ce**
**que** le sachet de captage (18) est réalisé soit en un treillis composé de métal ou d'un alliage métallique soit en un treillis en plastique.

3. Dispositif de filtrage selon la revendication 2,
**caractérisé en ce**
**que** le sachet de captage (18) est réalisé en un treillis en nitinol.

4. Dispositif de filtrage selon l'une des revendications précédentes,
**caractérisé en ce**
**qu'**à son extrémité prévu d'être glissée sur le sachet de captage (18), la douille protectrice (16) comporte un évasement (28).

5. Dispositif de filtrage selon l'une des revendications précédentes,
**caractérisé en ce**
**que** la douille protectrice (16) est conformée de façon flexible.

6. Dispositif de filtrage selon l'une des revendications précédentes,
**caractérisé en ce**
**que** l'extrémité (22) du sachet de captage (18) est collée ou soudée au corps de filtre (12) ou assemblé au corps de filtre (12) moyennant un organe de raccord (24).

7. Dispositif de filtrage selon l'une des revendications précédentes,
**caractérisé en ce**
**que** le corps de filtre (12) comporte un point de repère pour le repérage de la position du dispositif de filtrage (10) à l'intérieur du système vasculaire.

8. Dispositif de filtrage selon l'une des revendications précédentes,
**caractérisé en ce**
**que** le corps de filtre tubulaire (12) est traversé intérieurement par un fil de guidage (14).

9. Dispositif de filtrage selon l'une des revendications précédentes,
**caractérisé en ce que** le diamètre maxi. du dispositif de filtrage (10) est choisi, en état non-dilaté du sachet de captage (18), de manière à permettre le guidage du dispositif de filtrage (10) à l'intérieur d'un cathéter.

10. Dispositif de filtrage selon l'une des revendications précédentes,
**caractérisé en ce**
**que** le dispositif de filtrage (10) est solidaire d'un cathéter à ballon.

11. Dispositif de filtrage selon l'une des revendications 1 à 10,
**caractérisé en ce**
**que** le dispositif de filtrage (10) est solidaire d'un système de stents auto-dilatants (26).
